# FASCICULE DE BREVET EUROPEEN

(11) **EP 1 070 083 B1**
(45) Date de publication et mention de la délivrance du brevet: **04.01.2006**
(21) Numéro de dépôt: 99911890.4
(22) Date de dépôt: 07.04.1999
(51) Int. Cl.: C07K 5/023, A61K 38/06

(54) **FORMES CRISTALLINES DU 1S-[1ALPHA(2S*,3R*),9ALPHA]-6,10-DIOXO-N- (2-ETHOXY-5-OXO-TETRAHYDRO-3-FURANYL)-9-[[(1-ISOQUINOLYL)CARBONYL]- AMINO]OCTAHYDRO-6H-PIRIDAZINO[1,2-A][1,2]DIAZEPINE-1-CARBOXAMIDE**
KRISTALLINE FORMEN VON 1S-[1ALPHA(2S*,3R*),9ALPHA]-6,10-DIOXO-N- (2-ETHOXY-5-OXO-TETRAHYDRO-3-FURANYL)-9-[[(1-ISOCHINOLYL)CARBONYL]- AMINO]OCTAHYDRO-6H-PIRIDAZINO[1,2-A][1,2]DIAZEPIN-1-CARBOXYAMID
CRYSTALLINE FORMS OF 1S- [1ALPHA (2S*,3R*), 9ALPHA]-6, 10-DIOXO-N- (2-ETHOXY-5 -OXO-TETRAHYDRO-3 -FURANYL) -9- [[(1-ISOQUINOLYL) CARBONYL]-AMINO] OCTAHYDRO-6H -PIRIDAZINO [1, 2-A][ 1,2] DIAZEPINE-1-CARBOXAMIDE

(30) Priorité: 08.04.1998 FR 9804367
(43) Date de publication de la demande: 24.01.2001
(73) Titulaire: Aventis Pharma S.A., 92160 Antony (FR)
(72) Inventeur: GODARD, Jean-Yves, F-93340 Le Raincy (FR); ROGNON, Valérie, F-93470 Coubron (FR)
(86) Numéro de dépôt international: PCT/FR1999/000799
(87) Numéro de publication internationale: WO 1999/052935

(56) Documents cités:
- WO-A-97/22619

## Description

La présente invention a pour objet deux nouvelles formes cristallines du 1S-[1alpha(2S*,3R*),9alpha]6,10-dioxo-N-(2-éthoxy-5-oxo-tétrahydro-3-furanyl)-9[[(1-isoquinolyl)carbonyl]amino]octahydro-6H-piridazino[1,2-a][1,2]diazepine-1-carboxamide (anhydre ou hydraté), leur procédé de préparation et les compositions pharmaceutiques les renfermant.

La demande de brevet WO9722619 décrit le 1S-[lalpha (2S*,3R*),9alpha] 6,10-dioxo-N-(2-éthoxy-5-oxo-tétrahydro-3-furanyl)-9[[(1-isoquinolyl)carbonyl]amino]octahydro-6H-piridazino[1,2-a][1,2]diazepine-1-carboxamide ainsi que ses sels pharmaceutiquement acceptables (produit 412f : Composé I) à titre d'inhibiteurs de l'enzyme de conversion de l'interleukine-1bêta :

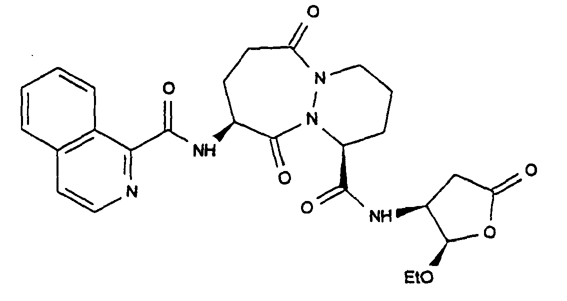

Le composé (I) tel que décrit et préparé dans cette demande WO9722619 se trouve sous une forme amorphe. Il présente principalement l'inconvénient d'être hygroscopique.

La préparation du composé (I) est effectuée de la manière suivante : Réaction d'amidification entre l'acide (1S,9S)9-(isoquinolin-1-oylamino)-6,10-dioxo-1,2,3,4,7,8,9,10-octahydro-6H-pyridazino-[1,2-a][1,2]-diazepine-1-carboxylique et le (3S,2S)3-allyloxycarbonylamino-2-benzyloxy-5-oxotétrahydrofurane en présence d'acide diméthylbarbiturique, de tétrakistriphénylphosphine palladium, de 1-hydroxybenzotriazole et de chlorhydrate de 1(3-diméthyl aminopropyl)-3-éthylcarbodiimide dans du chlorure de méthylène, du diméthylformamide ou un mélange de ces deux solvants. Le produit est purifié par chromatographie (Acétate d'éthyle/dichlorométhane) pour donner le composé (I) amorphe.

L'invention a pour objet de trouver une ou plusieurs nouvelles formes cristallines qui ne présentent pas les inconvénients de la forme amorphe.

Les formes solides, et notamment les produits pharmaceutiques, peuvent présenter plus d'une forme cristalline. C'est ce qu'on appelle le polymorphisme.

Les formes polymorphes d'une même molécule montrent en général des propriétés physiques différentes telles que la solubilité, l'hygroscopicité et la stabilité. Il faut noter qu'il n'existe pas pour le moment de méthodes permettant de prédire l'existence de tel ou tel polymorphe, ni de prédire leurs propriétés physiques.

L'obtention de nouvelles formes polymorphes de molécules ayant une activité thérapeutique présente un grand intérêt pour l'industrie pharmaceutique notamment du point de vue de leur préparation à une échelle industrielle, leur mise en oeuvre au sein de compositions pharmaceutiques, la recherche d'une meilleure stabilité et d'une meilleure biodisponibilité. (Byrn, S.R., Solid-State Chemistry of Drugs, New York, Academ. Press (1982) ; Kuhnert-Brandstatter, M, Thermomicroscopy In the Analysis of Pharmaceuticals, New York, Pergamon Press (1971) ; J. Halebian et al. J. Pharm. Science (1969) vol 58(8) 911 ; J. Halebian et al. J. Pharm. Science (1975) vol 64 (8) 1269-1288).

On entend par forme polymorphe toutes formes asolvatées d'une molécule cristallisée et pseudo-polymorphe toutes formes solvatées.

Les méthodes d'analyse des formes cristallines sont les suivantes :

Comportement thermique : il est déterminé par DSC (differential scanning calorimetry) (analyse calorimétrique différentielle) : 2 à 5 mg de substance à étudier sont pesées dans une capsule en aluminium scellée non hermétiquement. L'analyse est effectuée, sous balayage d'azote, de 25 à 350°C à une vitesse de montée en température de 20°C/mn.

IR (Infrarouge) : La substance à étudier est dispersée dans l'huile de paraffine liquide. L'analyse est effectuée sur un spectrophotomètre infrarouge à transformée de Fourier (FTIR) de 4000 à 600 cm⁻¹.

RX (diffraction des rayons X sur poudre) : La substance à étudier est répartie dans l'alvéole d'un porte échantillon en verre. L'analyse est effectuée par balayage de 2° à 38° (2 thêta) avec un pas de 0,02° et 1 seconde de comptage par pas. La source de rayons-X est un tube Cuivre (45kV, 30mA).

La demanderesse a mis en évidence deux nouvelles formes cristallines (forme A et forme B). La forme A qui est anhydre et la forme B qui est hydratée. La forme cristalline A présente, outre les avantages cités plus haut, une absence d'hygroscopicité (voir le test plus bas).

L'invention a donc d'abord pour objet une nouvelle forme cristalline du 1S-[1alpha(2S*,3R*),9alpha]6,10-dioxo-N-(2-éthoxy-5-oxo-tétrahydro-3-furanyl)-9[[(1-isoquinolyl) carbonyl]amino]octahydro-6H-piridazino[1,2-a][1,2] diazepine-1-carboxamide anhydre que l'on appelle forme A.
Cette forme A présente les caractéristiques suivantes :

Système cristallin : triclinique
a (Å) : 8,02 ; b (Å) : 9,21 ; c (Å) 17,70 ; alpha (degré) : 91,38 ; bêta (degré) : 93,62 ; gamma (degré) : 90,43 ; groupe d'espace P1 ; Z 2.

L'invention a également pour objet une nouvelle forme cristalline du 1S-[lalpha(2S*,3R*),9alpha]6,10-dioxo-N-(2-éthoxy-5-oxo-tétrahydro-3-furanyl)-9[[(1-isoquinolyl) carbonyl]amino]octahydro-6H-piridazino[1,2-a][1,2]diazepine-1-carboxamide hydraté que l'on appelle forme B.

### La forme A présente les caractéristiques suivantes :

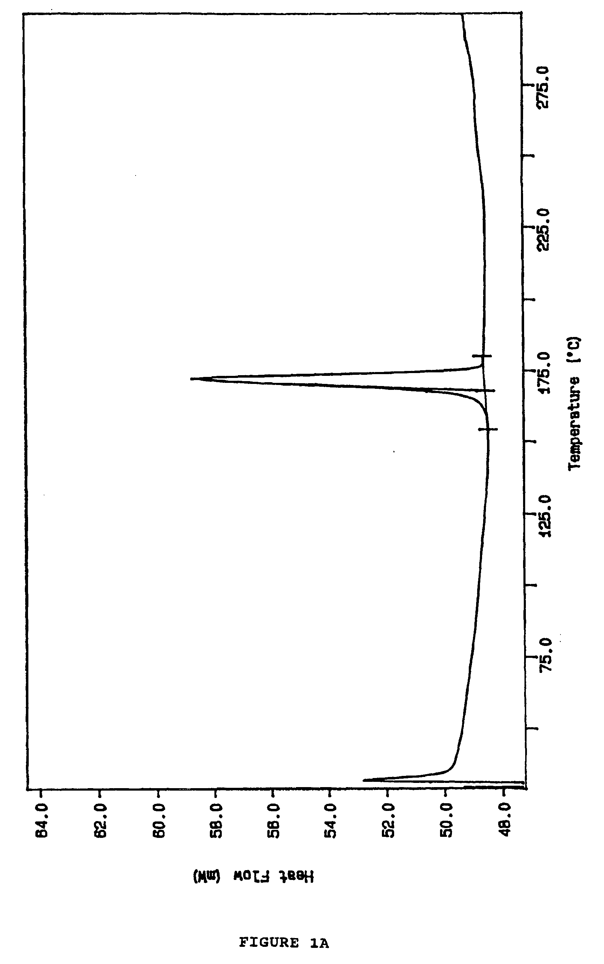

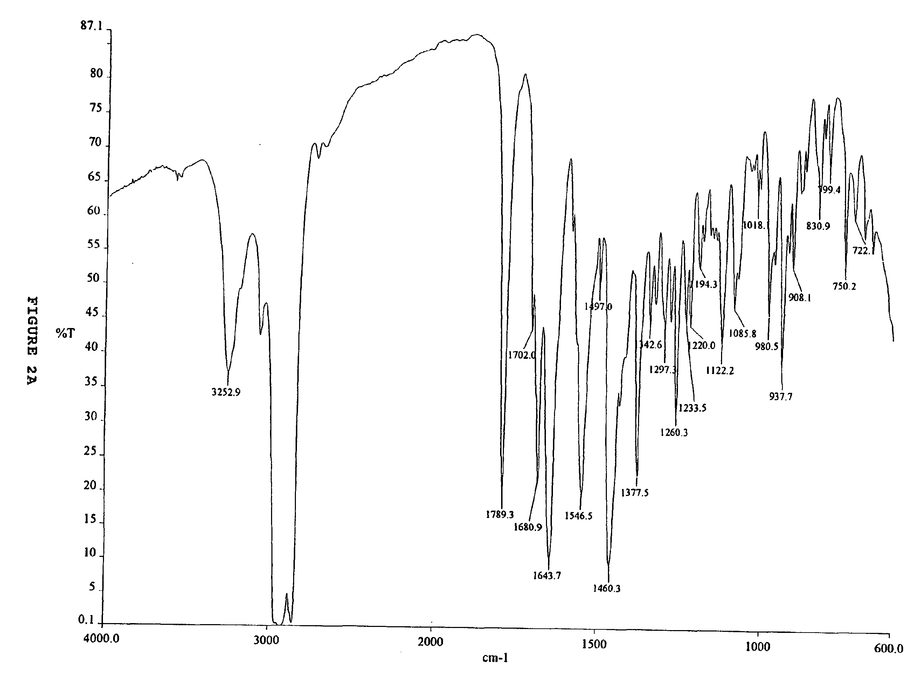

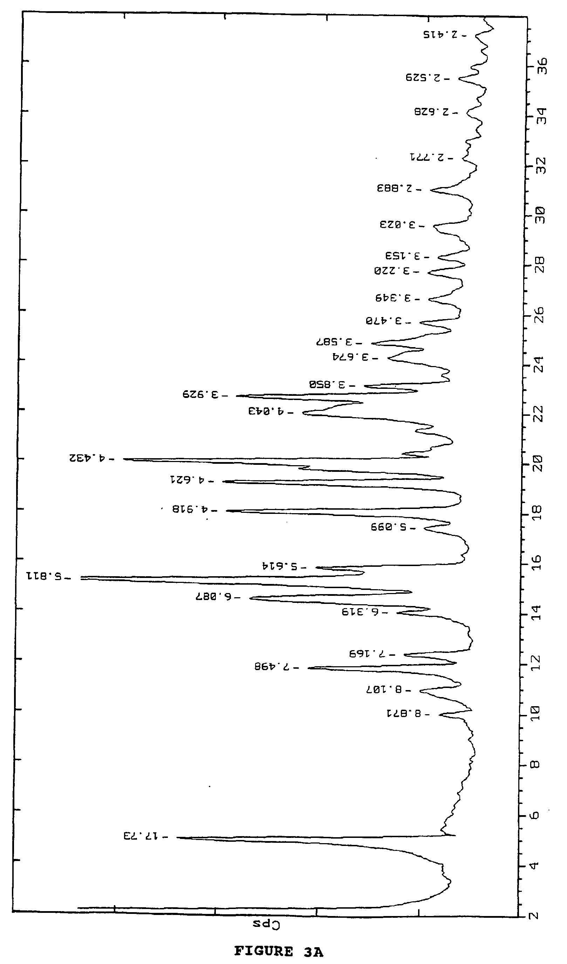

Le composé de formule (I) anhydre, tel que préparé par l'une des méthodes indiquées ci-dessous, possède une forme cristalline particulière (forme A). Les caractéristiques physiques sont décrites dans les figures 1A (DSC) 2A (IR) et 3A (RX).
DSC : Endotherme de fusion à partir de 168°C.
IR (nuiol, cm⁻¹) : 3253 ; 1789 ; 1702 ; 1681 ; 1644
RX (d,Å) : 17,73 ; 8,87 ; 8,11 ; 7,50 ; 7,17 ; 6,31 ; 6,09 ; 5,81.

### La forme B présente les caractéristiques suivantes :

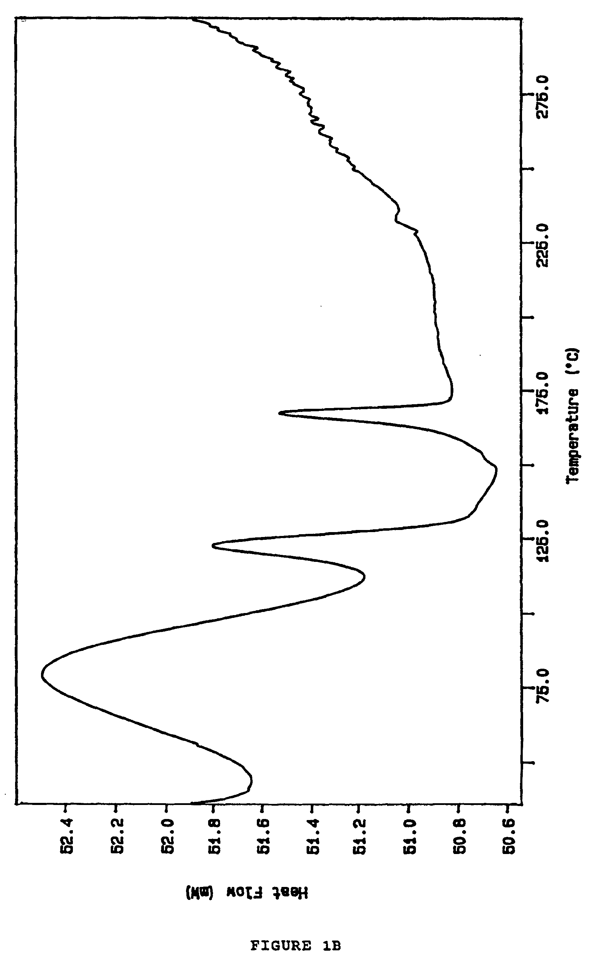

Le composé de formule (I) hydraté, tel que préparé par l'une des méthodes indiquées ci-dessous, possède une forme cristalline particulière (forme B). Les caractéristiques physiques sont décrites dans les figures 1B (DSC) 2B (IR) et 3B (RX).
DSC : Endotherme de déshydratation entre 50 et 110°C et entre 110 et 130°C. On observe un endotherme de fusion à partir de 162°C.
IR (nujol, cm⁻¹) : 3569 ; 3447 ; 3322 ; 1778 ; 1682 ; 1660
RX (d,Å) : 11,34 ; 10,80 ; 10,06 ; 7,59 ; 7,16 ; 6,71 ; 6,41 ; 6,11 ; 5,44.

L'invention a donc pour objet la forme A telle que définie précédemment ayant au moins l'une des caractéristiques suivantes et de préférence toutes les caractéristiques suivantes :
a) Endotherme de fusion à partir de 168°C,
b) spectre infrarouge présentant des absorptions caractéristiques à environ (nujol, cm⁻¹) : 3253 ; 1789 ; 1702 ; 1681 ; 1644,
c) diagramme de diffraction RX présentant distances interéticulaires caractéristiques égales à (d,Å) : 17,73 ; 8,87 ; 8,11 ; 7,50 ; 7,17 ; 6,31 ; 6,09 ; 5,81.

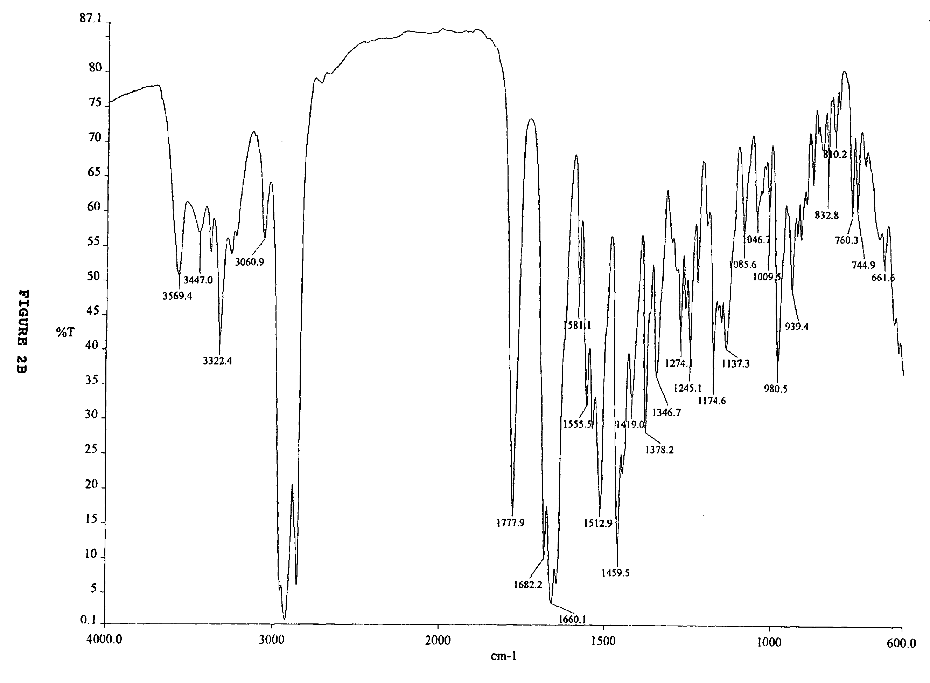

L'invention a plus particulièrement pour objet la forme A telle que définie précédemment présentant un spectre infrarouge substantiellement identique à celui de la figure 2A et un diagramme de diffraction RX substantiellement identique à celui de la figure 3A.

L'invention a également pour objet la forme B telle que définie précédemment ayant au moins l'une des caractéristiques suivantes et de préférence toutes ces caractéristiques suivantes :
a) Endotherme de déshydratation entre 50 et 110°C et entre 110 et 130°C et endotherme de fusion à partir de 162°C,
b) spectre infrarouge de la forme B présentant des absorptions caractéristiques à environ (nujol, cm⁻¹) : 3569 ; 3447 ; 3322 ; 1778 ; 1682 ; 1660,
c) diagramme de diffraction RX de la forme B présentant des distances interéticulaires caractéristiques égales à (d,Å) : 11,34 ; 10,80 ; 10,06 ; 7,59 ; 7,16 ; 6,71 ; 6,41 ; 6,11 ; 5,44.

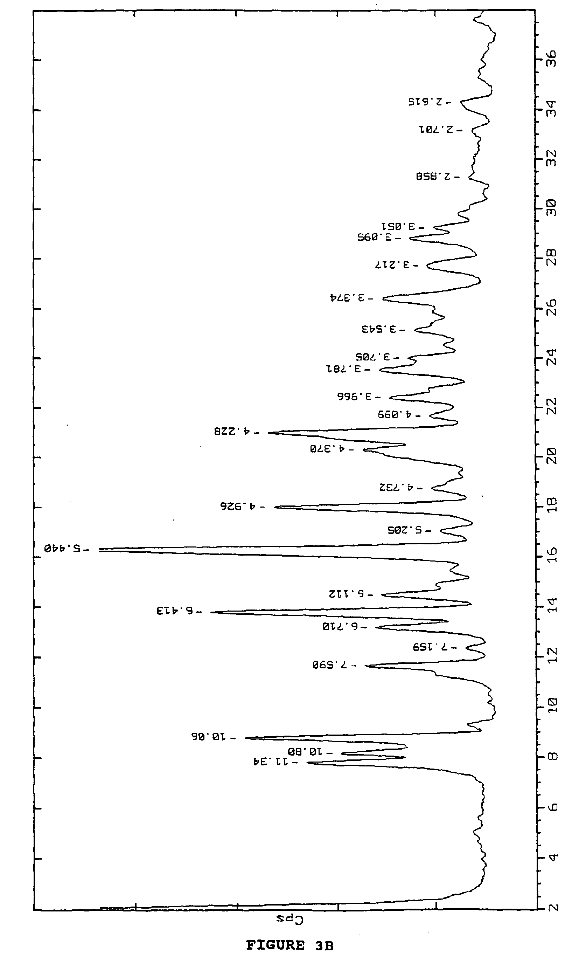

L'invention a également plus particulièrement pour objet la forme B telle que définie précédemment présentant un spectre infrarouge substantiellement identique à celui de la figure 2B et un diagramme de diffraction RX substantiellement identique à celui de la figure 3B.

La présente invention a également pour objet un procédé de préparation de la forme A ou B, caractérisé en ce que l'on dissout le composé (I) amorphe obtenu selon la méthode définie plus haut dans un solvant organique ou un mélange de ces solvants, et notamment à température ambiante, et on obtient après cristallisation la forme A ou B attendue.

L'obtention de la forme A s'effectue de préférence par cristallisation dans un alcool ou un éther et notamment l'éther isopropylique, le butanol ou l'isopropanol.

On obtient également la forme A par cristallisation dans un mélange de ces solvants, notamment dans le mélange éthanol/éther isopropylique.

L'obtention de la forme B s'effectue tout particulièrement par cristallisation dans le toluène.

L'obtention des formes A ou B attendues peut être, le cas échéant, initiée par un ensemencement de la solution avec quelques cristaux respectivement de forme A ou B.

L'isolement des formes A ou B s'effectue selon les méthodes connues de l'homme du métier.

Les formes cristallines A ou B du composé de formule (I) présentent les mêmes activités thérapeutiques que celles décrites pour le composé (I) amorphe dans la demande WO97/22169 et WO95/35308.

Elles présentent une activité inhibitrice de l'ICE (enzyme de conversion de l'interleukine-1bêta) et sont particulièrement utiles dans le traitement des maladies inflammatoires, auto-immunes et neurodégénératives.

L'invention a donc pour objet les formes cristallines A ou B telles que décrites précédemment à titre de médicament.

Les formes cristallines A ou B du composé de formule (I) peuvent être utilisées par voie orale, parentérale, topique par inhalation, ou via implants. Ils peuvent être prescrits sous forme de comprimés simples ou dragéifiés, de gélules, de granulés, de suppositoires, d'ovules, de préparations injectables, de pommades, de crèmes, de gels, de microsphères, d'implants, de patches, lesquels sont préparés selon les méthodes usuelles.

Les formes cristallines A ou B du composé de formule (I) peuvent être mélangées avec les excipients, diluants et tous véhicules connus de l'homme du métier pour la fabrication de compositions pharmaceutiques. A titre d'exemple d'excipients habituellement employés dans ces compositions pharmaceutiques on peut citer le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention s'étend ainsi aux compositions pharmaceutiques renfermant comme principe actif au moins l'une des formes cristallines A ou B du composé de formule (I) telles que définies ci-dessus et un ou plusieurs excipients, diluants ou supports pharmaceutiquement acceptables.

L'invention a également pour objet l'application des formes cristallines A ou B du composé de formule (I) telles que définies ci-dessus pour la préparation d'un médicament destiné à inhiber l'activité ICE (enzyme de conversion de l'interleukine-lbêta).

Les exemples suivants illustrent l'invention sans toutefois la limiter.

### EXEMPLE 1 : Obtention de la forme A par cristallisation dans le n-butanol.

A 300 mg composé I amorphe (obtenu selon la méthode décrite dans la demande WO9722619) on ajoute 1,5 ml de n-butanol et agite à température ambiante 2 heures 15. On observe la cristallisation du produit que l'on essore et sèche à 20°C sous 4mbars. On obtient 160 mg du composé de formule (I) anhydre (forme A).

### EXEMPLE 2 : Obtention de la forme A par cristallisation dans l'isopropanol.

A 300 mg composé I amorphe (obtenu selon la méthode décrite dans la demande WO9722619) on ajoute 1,5 ml d'isopropanol et agite à température ambiante 16 heures. On observe la cristallisation du produit que l'on essore et sèche à 20°C sous 4mbars. On obtient 166 mg du composé de formule (I) anhydre (forme A).

### EXEMPLE 3 : Obtention de la forme B par cristallisation dans le toluène.

A 300 mg composé I amorphe (obtenu selon la méthode décrite dans la demande WO9722619) on ajoute 1,5 ml de toluène et agite à température ambiante 16 heures. On observe la cristallisation du produit que l'on essore et sèche à 20°C sous 4mbars. On obtient le composé de formule (I) hydraté (forme B).

### EXEMPLE 4 : Obtention de la forme A par cristallisation dans un mélange éther isopropylique/éthanol.

A 11,9 g de composé (I) amorphe, on ajoute, sous azote et agitation, 23,8 ml d'éthanol absolu et chauffe la suspension à 60°±2°C pour obtenir une solution. On refroidit à 40°±2°C, amorce la cristallisation et maintient 1 heure à cette température. On ajoute ensuite 119 ml d'éther isopropylique en 15 minutes à 40°±2°C et maintient 15 minutes à cette température. On refroidit à 20°±2°C en 15 minutes, maintient 1 heure dans ces conditions, puis refroidit à nouveau à 0°,+5°C en 15 minutes et maintient 2 heures. On essore, lave avec de l'éther isopropylique, sèche sous pression réduite à 20°C pendant 12 heures et obtient 11,3 g de composé de formule (I) anhydre (forme A).

### Courbe d'hygroscopicité (ou isotherme de sorption d'eau)

Les isothermes de sorption d'eau ont été obtenus à l'aide d'un appareil Dynamic Vapor Sorption DVS 1 (Surface Measurements System Ltd) à température constante et sous atmosphère d'azote plus ou moins hydratée. Le degré d'humidité relative (HR) de l'atmosphère a été modifié par paliers. Le passage d'un palier au suivant s'effectue lorsque le poids de l'échantillon à analyser a atteint une valeur constante.

| RH (%) | AMORPHE | | FORME A ANHYDRE | | FORME B HYDRATEE | |
|---|---|---|---|---|---|---|
| | Sorption | Désorption | Sorption | Désorption | Sorption | Désorption |
| 0,0 | 0,00 | -0,20 | 0,00 | 0,11 | 0,00 | -0,11 |
| 12,0 | 0,67 | 1,48 | 0,03 | 0,12 | 1,41 | 1,42 |
| 21,0 | 0,97 | 2,09 | 0,05 | 0,14 | 2,36 | 2,33 |
| 32,0 | 1,29 | 2,58 | 0,07 | 0,19 | 3,23 | 3,14 |
| 43,0 | 1,60 | 2,94 | 0,09 | 0,21 | 3,88 | 3,75 |
| 51,0 | 1,83 | 3,19 | 0,11 | 0,22 | 4,26 | 4,13 |
| 57,0 | 2,03 | 3,35 | 0,13 | 0,21 | 4,53 | 4,40 |
| 66,0 | 2,37 | 3,61 | 0,14 | 0,21 | 4,92 | 4,75 |
| 75,0 | 2,90 | 3,90 | 0,14 | 0,21 | 5,27 | 5,08 |
| 80,0 | 3,71 | 4,12 | 0,16 | 0,21 | 5,45 | 5,30 |
| 90,0 | 5,11 | 4,94 | 0,14 | 0,19 | 5,93 | 5,96 |
| 96,0 | 5,93 | 5,93 | 0,06 | 0,06 | 6,86 | 6,86 |

Pendant le cycle de sorption la forme A n'est pas hygroscopique (< 0,2 %). La non hygroscopicité a été confirmée en laissant un échantillon 7 jours dans une chambre à 96 % d'humidité relative (à 32°C). On a observé une absorption d'eau d'environ 0,15 %. La forme hydrate (forme B) est plus hygroscopique (7 % d'eau) dans les mêmes conditions. Il en est de même en ce qui concerne la forme amorphe.

## Revendications

1. Forme cristalline du 1S-[1alpha(2S*,3R*),9alpha]6,10-dioxo-N-(2-éthoxy-5-oxo-tétrahydro-3-furanyl)-9[[(1-isoquinolyl)carbonyl]amino]octahydro-6H-piridazino[1,2-a][1,2]diazepine-1-carboxamide anhydre.

2. Forme cristalline du 1S-[1alpha(2S*,3R*),9alpha]6,10-dioxo-N-(2-éthoxy-5-oxo-tétrahydro-3-furanyl)-9[[(1-isoquinolyl)carbonyl]amino]octahydro-6H-piridazino[1,2-a][1,2]diazepine-1-carboxamide anhydre selon la revendication 1 ayant au moins l'une des caractéristiques suivantes :
a) Endotherme de fusion à partir de 168°C,
b) spectre infrarouge présentant des absorptions caractéristiques à (nujol, cm⁻¹) : 3253 ; 1789 ; 1702 ; 1681 ; 1644,
c) diagramme de diffraction RX présentant distances interéticulaires caractéristiques égales à (d,Å) : 17,73 ; 8,87 ; 8,11 ; 7,50 ; 7,17 ; 6,31 ; 6,09 ; 5,81.

3. Forme cristalline du 1S-[1alpha(2S*,3R*),9alpha]6,10-dioxo-N-(2-éthoxy-5-oxo-tétrahydro-3-furanyl)-9[[(1-isoquinolyl)carbonyl]amino]octahydro-6H-piridazino[1,2-a][1,2]diazepine-1-carboxamide anhydre telle que définie à la revendication 2 présentant les caractéristiques a, b et c.

4. Forme cristalline du 1S-[lalpha(2S*,3R*),9alpha]6,10-dioxo-N-(2-éthoxy-5-oxo-tétrahydro-3-furanyl)-9[[(1-isoquinolyl)carbonyl]amino]octahydro-6H-piridazino[1,2-a][1,2]diazepine-1-carboxamide anhydre telle que définie à la revendication 2 ou 3, présentant :
a) un spectre infrarouge présentant des absorptions caractéristiques à (nujol, cm⁻¹) : 3253 ; 1789 ; 1702 ; 1681 ; 1644 ; 1546 ; 1497 ; 1460 ; 1377 ; 1343 ; 1297 ; 1260 ; 1233 ; 1220 ; 1194 ; 1122 ; 1086 ; 1018 ; 980 ; 938 ; 908 ; 831 ; 799 ; 750 ; 722, et
b)un diagramme de diffraction RX présentant des distances interéticulaires caractéristiques égales à (d, Å) : 17,73 ; 8,87 ; 8,11 ; 7,50 ; 7,17 ; 6,32 ; 6,09 ; 5,81 ; 5,61 ; 5,10 ; 4,92 ; 4,62 ; 4,43 ; 4,04 ; 3,93 ; 3,85 ; 3,67 ; 3,59 ; 3,47 ; 3,35 ; 3,22 ; 3,15 ; 3,02 ; 2,88 ; 2,77 ; 2,63 ; 2,53 ; 2,41.

5. Forme cristalline du 1S-[1alpha(2S*,3R*),9alpha]6,10-dioxo-N-(2-éthoxy-5-oxo-tétrahydro-3-furanyl)-9[[(1-isoquinolyl)carbonyl]amino]octahydro-6H-piridazino[1,2-a][1,2]diazepine-1-carboxamide hydraté.

6. Forme cristalline du 1S-[1alpha(2S*,3R*),9alpha]6,10-dioxo-N-(2-éthoxy-5-oxo-tétrahydro-3-furanyl)-9[[(1-isoquinolyl)carbonyl]amino]octahydro-6H-piridazino[1,2-a][1,2]diazepine-1-carboxamide hydraté selon la revendication 5, ayant au moins l'une des caractéristiques suivantes :
a) Endotherme de déshydratation entre 50 et 110°C et entre 110 et 130°C et endotherme de fusion à partir de 162°C,
b) spectre infrarouge présentant des absorptions caractéristiques à (nujol, cm⁻¹) : 3569 ; 3447 ; 3322 ; 1778 ; 1682 ; 1660,
c) diagramme de diffraction RX présentant des distances interéticulaires caractéristiques égales à (d,Å) : 11,34 ; 10,80 ; 10,06 ; 7,59 ; 7,16 ; 6,71 ; 6,41 ; 6,11 ; 5,44.

7. Forme cristalline du 1S-[1alpha(2S*,3R*),9alpha]6,10-dioxo-N-(2-éthoxy-5-oxo-tétrahydro-3-furanyl)-9[[(1-isoquinolyl)carbonyl]amino]octahydro-6H-piridazino[1,2-a][1,2]diazepine-1-carboxamide hydraté telle que définie à la revendication 6, présentant les caractéristiques a, b et c.

8. Forme cristalline du 1S-[lalpha(2S*,3R*),9alpha]6,10-dioxo-N-(2-éthoxy-5-oxo-tétrahydro-3-furanyl)-9[[(1-isoquinolyl)carbonyl]amino]octahydro-6H-piridazino[1,2-a][1,2] diazepine-1-carboxamide hydraté telle que définie à la revendication 6 ou 7, présentant :
a)un spectre infrarouge présentant des absorptions caractéristiques à (nujol, cm⁻¹) : 3569 ; 3447 ; 3322 ; 3061 ; 1778 ; 1682 ; 1660 ; 1581 ; 1555 ; 1513 ; 1459 ; 1419 ; 1378 ; 1347 ; 1274 ; 1245 ; 1175 ; 1137 ; 1086 ; 1047 ; 1009 ; 980 ; 939 ; 833 ; 810 ; 760 ; 745 ; 662, et
b) un diagramme de diffraction RX présentant des distances interéticulaires caractéristiques égales à (d, Å) : 11,34 ; 10,80 ; 10,06 ; 7,59 ; 7,16 ; 6,71 ; 6,41 ; 6,11 ; 5,44 ; 5,20 ; 4,93 ; 4,73 ; 4,37 ; 4,23 ; 4,10 ; 3,97 ; 3,78 ; 3,70 ; 3,54 ; 3,37 ; 3,22 ; 3,09 ; 3,05 ; 2,86 ; 2,70 ; 2,61.

9. Procédé de préparation des formes cristallines telles que définies à l'une quelconque des revendications 1 à 8, **caractérisé en ce qu'**on mélange le 1S-[lalpha(2S*,3R*),9alpha] 6,10-dioxo-N-(2-éthoxy-5-oxo-tétrahydro-3-furanyl)-9[[(1-isoquinolyl)carbonyl]amino]octahydro-6H-piridazino[1,2-a][1,2] diazepine-1-carboxamide amorphe dans un solvant organique approprié ou un mélange de ces solvants, notamment à température ambiante, et on obtient après cristallisation la forme cristalline attendue.

10. Procédé selon la revendication 9, de préparation de la forme cristalline telle que définie à l'une quelconque des revendications 1 à 4, **caractérisé en ce que** le solvant est un éther et notamment l'éther isopropylique.

11. Procédé, selon la revendication 9, de préparation de la forme cristalline telle que définie à l'une quelconque des revendications 1 à 4, **caractérisé en ce que** le solvant est un alcool et notamment le n-butanol ou l'isopropanol.

12. Procédé, selon la revendication 9, de préparation de la forme cristalline telle que définie à l'une quelconque des revendications 1 à 4, **caractérisé en ce que** la cristallisation s'effectue dans un mélange d'alcool et d'éther et notamment dans le mélange éthanol/éther isopropylique.

13. Procédé, selon la revendication 9, de préparation de la forme cristalline telle que définie à l'une quelconque des revendications 5 à 8, **caractérisé en ce que** le solvant est le toluène.

14. A titre de médicament les formes cristallines telles que définies aux revendications 1 à 8.

15. Compositions pharmaceutiques renfermant au moins un médicament tel que défini à la revendication 13 et un ou plusieurs excipients, diluants ou supports pharmaceutiquement acceptables.

16. Application des formes cristallines telles que définies à l'une quelconque des revendications 1 à 8, pour la préparation d'un médicament destiné à inhiber l'activité ICE (enzyme de conversion de l'interleukine-1bêta).

## Patentansprüche

1. Kristalline Form von wasserfreiem 1S-[1-alpha-(2S*,3R*)-9-alphal-6,10-Dioxo-N-(2-ethoxy-5-Oxo-tetrahydro-3-furanyl)-9-{[(1-isochinolyl)-carbonyl]-amino}-octahydro-6H-pyridazino[1,2-a]-[1,2]-diazepin-1-carboxamid.

2. Kristalline Form von wasserfreiem 1S-[1-alpha-(2S*,3R*)-9-alpha]-6,10-Dioxo-N-(2-ethoxy-5-oxo-tetrahydro-3-furanyl)-9-{[(1-isochinolyl)-carbonyl]-amino}-octahydro-6H-pyridazino[1,2-a]-[1,2]-diazepin-1-carboxamid nach Anspruch 1 mit mindestens einer der folgenden Charakteristiken:
a) Schmelz-Endotherme ausgehend von 168 °C,
b) Infrarot-Spektrum, aufweisend die Absorptionscharakteristiken bei (Nujol, cm⁻¹): 3253 ; 1789 ; 1702 ; 1681 ; 1644,
c) Diffraktions-Diagramm RX, aufweisend die charakteristischen interetikulären Abstände gleich (d, Å) : 17,73 ; 8,87 ; 8,11 ; 7,50 ; 7,17 ; 6,31 ; 6,09 ; 5,81.

3. Kristalline Form von wasserfreiem 1S-[1-alpha-(2S*,3R*)-9-alpha]-6,10-Dioxo-N-(2-ethoxy-5-oxo-tetrahydro-3-furanyl)-9-{[(1-isochinolyl)-carbonyl]-amino}-octahydro-6H-pyridazino[1,2-a]-[1,2]-diazepin-1-carboxamid wie in Anspruch 2 definiert, aufweisend die Charakteristiken a, b und c.

4. Kristalline Form von wasserfreiem 1S-[1-alpha-(2S*,3R*)-9-alpha]-6,10-Dioxo-N-(2-ethoxy-5-oxo-tetrahydro-3-furanyl)-9-{[(1-isochinolyl)-carbonyl]-amino}-octahydro-6H-pyridazino[1,2-a]-[1,2]-diazepin-1-carboxamid wie in Anspruch 2 oder 3 definiert, aufweisend:
a) ein Infrarot-Spektrum, aufweisend die charakteristischen Absorptionen bei (Nujol, cm⁻¹): 3253 ; 1789 ; 1702 ; 1681 ; 1644 ; 1546 ; 1497 ; 1460 ; 1377 ; 1343 ; 1297 ; 1260 ; 1233 ; 1220 ; 1194 ; 1122 ; 1086 ; 1018 ; 980 ; 938 ; 908 ; 831 ; 799 ; 750 ; 722 und
b) ein Diffraktions-Diagramm RX, aufweisend die charakteristischen interetikulären Abstände gleich (d, Å) : 17,73 ; 8,87 ; 8,11 ; 7,50 ; 7,17 ; 6,32 ; 6,09 ; 5,81 ; 5,61 ; 5,10 ; 4,92 ; 4,62 ; 4,43 ; 4,04 ; 3,93 ; 3,85 ; 3,67 ; 3,59 : 3,47 ; 3,35 ; 3,22 ; 3,15 ; 3,02 ; 2,88 ; 2,77 ; 2,63 ; 2,53 ; 2,41.

5. Kristalline Form von hydratisiertem 1S-[1-alpha-(2S*,3R*)-9-alpha]-6,10-Dioxo-N-(2-ethoxy-5-oxo-tetrahydro-3-furanyl)-9-{[(1-isochinolyl)-carbonyl]-amino}-octahydro-6H-pyridazino[1,2-a]-[1,2]-diazepin-1-carboxamid.

6. Kristalline Form von hydratisiertem 1S-[1-alpha-(2S*,3R*)-9-alpha]-6,10-Dioxo-N-(2-ethoxy-5-oxo-tetrahydro-3-furanyl)-9-{[(1-isochinolyl)-carbonyl]-amino}-octahydro-6H-pyridazino[1,2-a]-[1,2]-diazepin-1-carboxamid nach Anspruch 5, mit mindestens einer der folgenden Charakteristiken:
a) Dehydratisierungs-Endotherme zwischen 50 °C und 110 °C und zwischen 110 °C und 130 °C und Schmelz-Endotherme ausgehend von 162 °C,
b) Infrarot-Spektrum, aufweisend die charakteristischen Absorptionen bei (Nujol, cm⁻¹): 3569 ; 3447 ; 3322 ; 1778 ; 1682 ; 1660,
c) Diffraktions-Diagramm RX, aufweisend die charakteristischen interetikulären Abstände gleich (d, Å) : 11,34 ; 10,80 ; 10,06 ; 7,59 ; 7,16 ; 6,71 ; 6,41; 6,11 ; 5,44.

7. Kristalline Form von hydratisiertem 1S-[1-alpha-(2S*,3R*)-9-alpha]-6,10-Dioxo-N-(2-ethoxy-5-oxo-tetrahydro-3-furanyl)-9-{[(1-isochinolyl)-carbonyl]-amino}-octahydro-6H-pyridazino[1,2-a]-[1,2]-diazepin-1-carboxamid wie in Anspruch 6 definiert, aufweisend die Charakteristiken a, b und c.

8. Kristalline Form von hydratisiertem 1S-[1-alpha-(2S*,3R*)-9-alpha]-6,10-Dioxo-N-(2-ethoxy-5-oxo-tetrahydro-3-furanyl)-9-{[(1-isochinolyl)-carbonyl]-amino}-octahydro-6H-pyridazino[1,2-a]-[1,2]-diazepin-1-carboxamid wie in Anspruch 6 oder 7 definiert, aufweisend:
a) ein Infrarot-Spektrum, aufweisend die charakteristischen Absorptionen bei (Nujol, cm⁻¹): 3569 ; 3447 ; 3322 ; 3061 ; 1778 ; 1682 ; 1660 ; 1581 ; 1555 ; 1513 ; 1459 ; 1419 ; 1378 ; 1347 ; 1274 ; 1245 ; 1175 ; 1137 ; 1086 ; 1047 ; 1009 ; 980 ; 939 ; 833 ; 810 ; 760 ; 745 ; 662 und
b) ein Diffraktions-Diagramm RX, aufweisend die charakteristischen interetikulären Abstände gleich (d, Å) : 11,34 ; 10,80 ; 10,06 ; 7,59 ; 7,16 ; 6,71 ; 6,41 ; 6,11 ; 5,44 ; 5,20 ; 4,93 ; 4,73 ; 4,37 ; 4,23 ; 4,10 ; 3,97 ; 3,78 ; 3,70 ; 3,54 ; 3,37 ; 3,22 ; 3,09 ; 3,05 ; 2,86 ; 2,70 ; 2,61.

9. Verfahren zur Herstellung der kristallinen Form wie in irgendeinem der Ansprüche 1 bis 8 definiert, **dadurch gekennzeichnet, daß** man das amorphe 1S-[1-alpha-(2S*,3R*)-9-alpha]-6,10-Dioxo-N-(2-ethoxy-5-oxo-tetrahydro-3-furanyl)-9-{[(1-isochinolyl)-carbonyl]-amino}-octahydro-6H-pyridazino[1,2-a]-[1,2]-diazepin-1-carboxamid in einem geeigneten organischen Lösungsmittel oder einer Mischung dieser Lösungsmittel, insbesondere bei Umgebungstemperatur, mischt und nach der Kristallisation die erwartete kristalline Form erhält.

10. Verfahren nach Anspruch 9 zur Herstellung der kristallinen Form wie in irgendeinem der Ansprüche 1 bis 4 definiert, **dadurch gekennzeichnet, daß** das Lösungsmittel ein Ether ist, und insbesondere Isopropylether.

11. Verfahren nach Anspruch 9 zur Herstellung der kristallinen Form wie in irgendeinem der Ansprüche 1 bis 4 definiert, **dadurch gekennzeichnet, daß** das Lösungsmittel ein Alkohol ist, und insbesondere n-Butanol oder Isopropanol.

12. Verfahren nach Anspruch 9 zur Herstellung der kristallinen Form wie in irgendeinem der Ansprüche 1 bis 4 definiert, **dadurch gekennzeichnet, daß** die Kristallisation in einer Mischung von Alkohol und Ether und insbesondere in der Mischung Ethanol/Isopropylether durchgeführt wird.

13. Verfahren nach Anspruch 9 zur Herstellung der kristallinen Form wie in irgendeinem der Ansprüche 5 bis 8 definiert, **dadurch gekennzeichnet, daß** das Lösungsmittel Toluol ist.

14. Als Arzneimittel die kristallinen Formen wie in den Ansprüchen 1 bis 8 definiert.

15. Pharmazeutische Zusammensetzungen, umfassend mindestens ein Arzneimittel wie in Anspruch 13 definiert und einen oder mehrere pharmazeutisch akzeptable Füllstoffe, Verdünnungsmittel oder Trägerstoffe.

16. Verwendung der kristallinen Formen wie in irgendeinem der Ansprüche 1 bis 8 definiert zur Herstellung eines Arzneimittels, das vorgesehen ist für die Inhibierung der Aktivität ICE (Interleukin-1-beta-Konversions-Enzyme).

## Claims

1. Crystalline form of anhydrous 1S-[1α(2S*,3R*), 9α]-6,10-dioxo-N-(2-ethoxy-5-oxo-tetrahydro-3-furanyl)-9-[[(1-isoquinolyl)carbonyl]amino]-octahydro-6H-pyridazino[1,2-a][1,2]diazepine-1-carboxamide.

2. Crystalline form of anhydrous 1S-[1α(2S*,3R*), 9α]-6,10-dioxo-N-(2-ethoxy-5-oxo-tetrahydro-3-furanyl)-9-[[(1-isoquinolyl)carbonyl]amino]-octahydro-6H-pyridazino[1,2-a][1,2]diazepine-1-carboxamide according to Claim 1 having at least one of the following characteristics:
a) melting endotherm from 168°C,
b) infrared spectrum exhibiting characteristic absorptions at (nujol, cm⁻¹) : 3253, 1789, 1702, 1681, 1644,
c) X-ray diffraction diagram exhibiting characteristic lattice spacings equal to (d, Å): 17.73, 8.87, 8.11, 7.50, 7.17, 6.31, 6.09, 5.81.

3. Crystalline form of anhydrous 1S-[1α(2S*,3R*), 9α]-6,10-dioxo-N-(2-ethoxy-5-oxo-tetrahydro-3-furanyl)-9-[[(1-isoquinolyl)carbonyl]amino]-octahydro-6H-pyridazino[1,2-a][1,2]diazepine-1-carboxamide as defined in Claim 2 exhibiting the characteristics a, b and c.

4. Crystalline form of anhydrous 1S-[1α(2S*,3R*), 9α]-6,10-dioxo-N-(2-ethoxy-5-oxo-tetrahydro-3-furanyl)-9-[[(1-isoquinolyl)carbonyl]amino]-octahydro-6H-pyridazino[1,2-a][1,2]diazepine-1-carboxamide as defined in Claim 2 or 3, exhibiting:
a) an infrared spectrum exhibiting characteristic absorptions at (Nujol, cm⁻¹): 3253, 1789, 1702, 1681, 1644, 1546, 1497, 1460, 1377, 1343, 1297, 1260, 1233, 1220, 1194, 1122, 1086, 1018, 980, 938, 908, 831, 799, 750, 722, and
b) an X-ray diffraction diagram exhibiting characteristic lattice spacings equal to (d, Å): 17.73, 8.87, 8.11, 7.50, 7.17, 6.32, 6.09, 5.81, 5.61, 5.10, 4.92, 4.62, 4.43, 4.04, 3.93, 3.85, 3.67, 3.59, 3.47, 3.35, 3.22, 3.15, 3.02, 2.88, 2.77, 2.63, 2.53, 2.41.

5. Crystalline form of 1S-[1α(2S*,3R*), 9α]-6,10-dioxo-N-(2-ethoxy-5-oxo-tetrahydro-3-furanyl)-9-[[(1-isoquinolyl)carbonyl]amino]-octahydro-6H-pyridazino[1,2-a][1,2]diazepine-1-carboxamide hydrate.

6. Crystalline form of 1S-[1α(2S*,3R*), 9α]-6,10-dioxo-N-(2-ethoxy-5-oxo-tetrahydro-3-furanyl)-9-[[(1-isoquinolyl)carbonyl]amino]-octahydro-6H-pyridazino[1,2-a][1,2]diazepine-1-carboxamide hydrate according to Claim 5 having at least one of the following characteristics:
a) dehydration endotherm between 50 and 110°C and between 110 and 130°C and melting endotherm from 162°C,
b) infrared spectrum exhibiting characteristic absorptions at (Nujol, cm⁻¹): 3569, 3447, 3322, 1778, 1682, 1660,
c) X-ray diffraction diagram exhibiting characteristic lattice spacings equal to (d, Å): 11.34, 10.80, 10.06, 7.59, 7.16, 6.71, 6.41, 6.11, 5.44.

7. Crystalline form of 1S-[1α(2S*,3R*), 9α]-6,10-dioxo-N-(2-ethoxy-5-oxo-tetrahydro-3-furanyl)-9-[[(1-isoquinolyl)carbonyl]amino]-octahydro-6H-pyridazino[1,2-a][1,2]diazepine-1-carboxamide hydrate as defined in Claim 6 exhibiting the characteristics a, b and c.

8. Crystalline form of 1S-[1α(2S*,3R*), 9α]-6,10-dioxo-N-(2-ethoxy-5-oxo-tetrahydro-3-furanyl)-9-[[(1-isoquinolyl)carbonyl]amino]-octahydro-6H-pyridazino[1,2-a][1,2]diazepine-1-carboxamide hydrate as defined in Claim 6 or 7, exhibiting:
a) an infrared spectrum exhibiting characteristic absorptions at (Nujol, cm⁻¹): 3569, 3447, 3322, 3061, 1778, 1682, 1660, 1581, 1555, 1513, 1459, 1419, 1378, 1347, 1274, 1245, 1175, 1137, 1086, 1047, 1009, 980, 939, 833, 810, 760, 745, 662, and
b) an X-ray diffraction diagram exhibiting characteristic lattice spacings equal to (d, Å): 11.34, 10.80, 10.06, 7.59, 7.16, 6.71, 6.41, 6.11, 5.44, 5.20, 4.93, 4.73, 4.37, 4.23, 4.10, 3.97, 3.78, 3.70, 3.54, 3.37, 3.22, 3.09, 3.05, 2.86, 2.70, 2.61.

9. Process for the preparation of the crystalline forms as defined in any one of Claims 1 to 8, **characterized in that** amorphous 1S-[1α(2S*,3R*), 9α]-6,10-dioxo-N-(2-ethoxy-5-oxo-tetrahydro-3-furanyl)-9-[[(1-isoquinolyl)carbonyl]amino]-octahydro-6H-pyridazino[1,2-a][1,2]diazepine-1-carboxamide is mixed in an appropriate organic solvent or a mixture of these solvents, in particular at ambient temperature, and the expected crystalline form is obtained after crystallization.

10. Process according to Claim 9 for the preparation of the crystalline form as defined in any one of Claims 1 to 4, **characterized in that** the solvent is an ether and in particular isopropyl ether.

11. Process according to Claim 9 for the preparation of the crystalline form as defined in any one of Claims 1 to 4, **characterized in that** the solvent is an alcohol and in particular n-butanol or isopropanol.

12. Process according to Claim 9 for the preparation of the crystalline form as defined in any one of Claims 1 to 4, **characterized in that** the crystallization is carried out from a mixture of alcohol and of ether and in particular from the ethanol/isopropyl ether mixture.

13. Process according to Claim 9 for the preparation of the crystalline form as defined in any one of Claims 5 to 8, **characterized in that** the solvent is toluene.

14. As medicament, the crystalline forms as defined in Claims 1 to 8.

15. Pharmaceutical compositions including at least one medicament as defined in Claim 13 and one or more pharmaceutically acceptable excipients, diluents or vehicles.

16. Application of the crystalline forms as defined in any one of Claims 1 to 8 in the preparation of a medicament intended to inhibit ICE (interleukin-1β converting enzyme) activity.
